Europäisches Patentamt

(19) European Patent Office

Office européen des brevets

(11) EP 0 679 709 B1

## (12) FASCICULE DE BREVET EUROPEEN

(45) Date de publication et mention
de la délivrance du brevet:
**04.10.2001 Bulletin 2001/40**

(51) Int Cl.$^7$: **C10G 57/00**, C07C 1/04

(21) Numéro de dépôt: **95400888.4**

(22) Date de dépôt: **20.04.1995**

(54) **Procédé de conversion du gaz naturel en hydrocarbures**

Verfahren zur Umsetzung von Erdgas zu Kohlenwasserstoffen

Process for the conversion of natural gas into hydrocarbons

(84) Etats contractants désignés:
**DE GB IT NL SE**

(30) Priorité: **25.04.1994 FR 9405065**

(43) Date de publication de la demande:
**02.11.1995 Bulletin 1995/44**

(73) Titulaire: **INSTITUT FRANCAIS DU PETROLE**
**92502 Rueil-Malmaison (FR)**

(72) Inventeurs:
 • **Chaumette, Patrick**
  **F-78380 Bougival (FR)**
 • **Boucot, Pierre**
  **F-69360 Ternay (FR)**

(56) Documents cités:
 **EP-A- 0 142 888**

**Description**

La présente invention concerne un procédé de fabrication d'hydrocarbures essentiellement linéaires et saturés, comprenant de préférence une proportion importante d'hydrocarbures comprenant au moins 5 atomes de carbone par molécule ($C_5^+$), à partir du gaz naturel.

Le gaz naturel est une matière première fossile abondante constituée majoritairement de méthane mais contenant souvent des quantités importantes d'éthane et d'hydrocarbures $C_3^+$ (c'est-à-dire des hydrocarbures comportant au moins 3 atomes de carbone par molécule), principalement $C_2$-$C_4$ (c'est-à-dire des hydrocarbures comportant de 2 à 4 atomes de carbone par molécule), ainsi que d'autres constituants tels que l'eau ($H_2O$), le gaz carbonique ($CO_2$), l'azote ($N_2$), l'hydrogène sulfuré ($H_2S$) et l'hélium (He).

Le gaz naturel est généralement purifié, cette purification consistant entre autres à enlever au moins l'hydrogène sulfuré, l'eau et l'hélium.

Après purification, le gaz naturel, ayant une teneur en composé soufré de préférence inférieure à 10 ppm, contient principalement du méthane (par exemple 55-99 % en volume) mais aussi des hydrocarbures $C_2^+$, principalement $C_2$-$C_4$, et éventuellement de faibles quantités d'azote et/ou de dioxyde de carbone.

Par abus de langage, on appelle généralement gaz naturel indifféremment le gaz issu d'un gisement et n'ayant subi aucune purification préalable ou le gaz purifié obtenu après déshydration et désulfuration.

Il est connu de l'homme de métier que le gaz naturel peut être transformé en gaz de synthèse (c'est-à-dire en un mélange comprenant de l'oxyde de carbone, de l'hydrogène et éventuellement du dioxyde de carbone, ledit mélange étant noté ci-après CO-($CO_2$)-$H_2$). Cette transformation peut se faire par divers procédés tels que le réformage à l'eau, couramment effectué en deux étapes de réaction, ou l'oxydation partielle (Ulmann's Encyclopedia of Industrial Chemistry Volume A12, p.186-212).

Afin d'améliorer le rendement thermique (cest-à-dire le rapport entre la capacité calorifique du gaz obtenu après réformage ou oxydation partielle et la charge de gaz naturel), il a été proposé un procédé dit de reformage autotherme opérant en présence d'eau et d'air (Ulmann's Encyclopedia of Industrial Chemistry Volume A12, p. 202 ; demande de brevet français FR-A-2.679.217).

Il est également connu de l'homme de métier que ledit gaz de synthèse peut être converti en hydrocarbures en présence de catalyseur contenant au moins un métal de transition. Cette réaction est connue dans la littérature sous le nom de synthèse Fischer-Tropsch (Ulmann's Encyclopedia of Industrial Chemistry Volume A7, p.206). Le catalyseur habituellement utilisé pour ladite synthèse comprend généralement au moins un métal des groupes 6 à 11 tel que le fer, le ruthénium, le cobalt ou le nickel (Catalysis Letters vol.7, p.303, 1990). Les produits préparés par synthèse Fischer-Tropsch en présence dudit catalyseur métallique présentent une distribution très large en terme de poids moléculaire. Une proportion importante des hydrocarbures produits contient plus de 5 atomes de carbone par molécule (hydrocarbures $C_5^+$); de plus, lesdits hydrocarbures produits sont principalement des hydrocarbures essentiellement linéaires et saturés de hauts points d'ébullition, c'est-à-dire ayant des points d'ébullition situés au-delà du domaine des distillats moyens (fraction kérosène dont les points d'ébullition sont respectivement compris entre 140 et 300° C et fraction gas oil dont les points d'ébullition sont compris entre 180 et 370° C). Il est souvent avantageux de traiter au moins une partie des hydrocarbures produits lors de la synthèse Fischer-Tropsch dans un procédé catalytique d'hydrotraitement opéré en présence d'hydrogène. Le catalyseur utilisé dans ledit procédé contient préférentiellement au moins un métal des groupes 6 à 11 présentant une activité hydrogénante, ledit métal étant dispersé sur un support. Ledit procédé permet notamment de transformer les hydrocarbures à hauts points d'ébullition en produits plus légers et donc d'augmenter de manière significative le rendement en distillats moyens.

Toutefois, une combinaison de procédés comprenant la transformation du gaz naturel en gaz de synthèse, la synthèse Fischer-Tropsch et éventuellement le procédé d'hydrotraitement est également productrice d'hydrocarbures légers, saturés et insaturés, ayant au plus 4 atomes de carbone par molécule (hydrocarbures $C_4^-$), principalement $C_2$-$C_4$, difficilement valorisables. Le brevet US-A-4.587.008 décrit le recyclage d'au moins une partie desdits hydrocarbures à la production de gaz de synthèse.

Le procédé de la présente invention permet de convertir le gaz naturel en hydrocarbures essentiellement linéaires et saturés comprenant de préférence une proportion importante d'hydrocarbures $C_5^+$, tout en valorisant avantageusement les hydrocarbures $C_2^+$, principalement $C_2$-$C_4$, essentiellement linéaires et saturés, contenus dans le gaz naturel, ainsi qu'éventuellement au moins une partie des hydrocarbures $C_4^-$, principalement $C_2$-$C_4$, essentiellement linéaires et saturés, provenant de la synthèse Fischer-Tropsch et éventuellement du procédé éventuel d'hydrotraitement.

Le procédé de la présente invention est un procédé de préparation d'hydrocarbures essentiellement linéaires et saturés comprenant de préférence une proportion importante d'hydrocarbures $C_5^+$ à partir d'une charge comprenant essentiellement du gaz naturel, ledit procédé comprenant les étapes successives suivantes, les étapes (b) et (c) étant telles qu'elle peuvent être réalisées soit simultanément soit dans l'ordre (b) puis (c) soit dans l'ordre (c) puis (b) :

(a) la production de gaz de synthèse par traitement d'au moins une partie de ladite charge permettant l'obtention d'un effluent comprenant principalement de l'oxyde de carbone, de l'hydrogène et éventuellement du dioxyde de carbone,

(b) le mélange d'un effluent comprenant au moins la majeure partie de l'effluent obtenu à l'étape précédente et d'un effluent comprenant principalement au moins un hydrocarbure comportant de 2 à 4 atomes de carbone par molécule,

(c) le refroidissement d'un effluent comprenant au moins la majeure partie du mélange obtenu à l'étape précédente, permettant l'obtention d'un effluent refroidi,

(d) la transformation dans une zone de craquage de la majeure partie de l'effluent refroidi obtenu à l'étape précédente, permettant l'obtention d'un effluent comprenant des hydrocarbures insaturés,

(e) la séparation d'au moins la majeure partie de l'effluent obtenu à l'étape (d) en un effluent comprenant principalement de l'eau et en un effluent pratiquement exempt d'eau,

(f) la transformation d'au moins la majeure partie de l'effluent pratiquement exempt d'eau obtenu à l'étape (e) par synthèse Fischer-Tropsch, en présence d'un catalyseur de synthèse Fischer-Tropsch, permettant d'obtenir 1' effluent final.

Dans le cas où les étapes (b) et (c) sont réalisées simultanément, on procède donc, suite à l'étape (a), à une étape dans laquelle au moins la majeure partie de l'effluent obtenu à l'étape (a) est mélangée à un effluent comprenant principalement au moins un hydrocarbure $C_2$-$C_4$ et refroidi, permettant l'obtention d'un effluent refroidi.

Dans le cas où les étapes (b) et (c) sont réalisées dans l'ordre (b) puis (c), on procède donc, suite à l'étape (a) et avant l'étape (d), aux étapes successives suivantes :

(b) le mélange d'un effluent comprenant au moins la majeure partie de l'effluent obtenu à l'étape (a) et d'un effluent comprenant principalement au moins un hydrocarbure $C_2$-$C_4$,

(c) le refroidissement d'un effluent comprenant au moins la majeure partie du mélange obtenu à l'étape (b) permettant l'obtention d'un effluent refroidi.

Dans le cas où les étapes (b) et (c) sont réalisées dans l'ordre (c) puis (b), on procède donc, suite à l'étape (a) et avant l'étape (d), aux étapes successives suivantes :

(c) le refroidissement d'un effluent comprenant au moins la majeure partie du mélange obtenu à l'étape (a), permettant l'obtention d'un effluent refroidi,

(b) le mélange d'un effluent comprenant au moins la majeure partie de l'effluent refroidi obtenu à l'étape (b) d'un effluent comprenant principalement au moins un hydrocarbure $C_2$-$C_4$, éventuellement refroidi.

Dans le cas d'une réalisation préférée du procédé selon l'invention, l'effluent comprenant principalement au moins un hydrocarbure comportant de 2 à 4 atomes de carbone par molécule est de préférence au moins partiellement issu de l'un au moins des procédés suivants : un procédé de synthèse Fischer-Tropsch, un procédé d'hydrotraitement ; ledit effluent peut dans ce cas préféré être bien sûr un effluent du procédé selon l'invention.

Une des réalisations préférées du procédé selon l'invention comprend la séparation de l'effluent issu de l'étape (f) en un premier effluent contenant principalement des hydrocarbures essentiellement linéaires et saturés contenant de 2 à 4 atomes de carbone par molécule et en un second effluent contenant principalement des hydrocarbures essentiellement linéaires et saturés contenant au moins cinq atomes de carbone par molécule. Dans le cas d'une telle réalisation préférée, l'effluent comprenant principalement au moins un hydrocarbure $C_2$-$C_4$ de l'étape (b) comprend au moins la majeure partie dudit premier effluent obtenu à l'étape (f). Mais, dans le cas d'une telle réalisation préférée, on peut procéder aussi de préférence, à l'issue de l'étape (f), à l'hydrotraitement dudit second effluent ; dans ce cas, de manière encore plus préférée, l'effluent comprenant principalement au moins un hydrocarbure $C_2$-$C_4$ de l'étape (b) comprend au moins la majeure partie d'un effluent comprenant principalement des hydrocarbures $C_2$-$C_4$ issus dudit hydrotraitement.

Le procédé selon l'invention est de préférence effectué de façon telle que, préalablement à l'étape (a) du procédé selon l'invention, on procède à une séparation de la charge en une première fraction enrichie en méthane, c'est-à-dire comprenant au moins 85% molaire de méthane, de préférence au moins 90% molaire de méthane et de manière encore plus préférée au moins 95% molaire de méthane, et une seconde fraction enrichie en au moins un hydrocarbure $C_2^+$, principalement $C_2$-$C_4$, c'est-à-dire comprenant au moins 85% molaire d'hydrocarbure $C_2^+$, principalement $C_2$-$C_4$, de préférence au moins 90% molaire d'hydrocarbure $C_2^+$ et de manière encore plus préférée au moins 95% molaire d'hydrocarbure $C_2^+$, principalement $C_2$-$C_4$, de façon que la partie de ladite charge traitée à l'étape (a) soit principalement la majeure partie de ladite première fraction. Dans le cas d'un tel procédé préféré selon l'invention, l'effluent comprenant principalement au moins un hydrocarbure $C_2$-$C_4$ de l'étape (b) comprend au moins la majeure partie de ladite seconde fraction (contenant généralement une grande proportion d'éthane).

L'étape (d) selon l'invention de craquage d'hydrocarbures principalement $C_2$-$C_4$ permet de transformer lesdits hydrocarbures, essentiellement linéaires et saturés, et inertes dans la réaction de synthèse Fischer-Tropsch, en des hydrocarbures essentiellement insaturés qui constituent des initiateurs de chaîne dans ladite réaction (A.A. ADESINA, R.R. HUDGINS et P.L. SILVESTON, Applied Catalysis vol. 61, p. 295, 1990 ; P. CHAUMETTE, C. VERDON, A. KIENNEMANN, S. BOUJANA, A.C.S. Div. Pet. Chem. Vol. 37, n° 3, p. 833, April 1992). Il en découle, pour la réaction de synthèse Fischer-Tropsch, une augmentation de la sélectivité en hydrocarbures $C_5^+$ et une meilleure valorisation des hydrocarbures légers $C_1$-$C_4$.

Les différentes étapes pouvant être comprises dans une réalisation du procédé selon l'invention tel que décrit ci-dessus sont des étapes connues de l'homme du métier. Néanmoins, quelques précisions sont données ci-après.

L'étape (a) de production de gaz de synthèse est de préférence une étape d'oxydation partielle d'au moins une partie de la charge comprenant essentiellement du gaz naturel (combustible) en présence d'un carburant dans une chambre de combustion et comportant éventuellement au moins un organe d'injection de carburant complémentaire, de la vapeur d'eau pouvant être éventuellement ajoutée avec le carburant ou le combustible dans la chambre de combustion. La charge combustible enrichie en méthane peut éventuellement être mélangée à un effluent CO-$(CO_2)$-$H_2$.

Le comburant utilisé dans ladite étape peut être de l'oxygène pur, de l'oxygène mélangé à un gaz inerte tel que l'azote, de la vapeur, ou du gaz carbonique.

Il est également possible d'utiliser pour ladite étape (a) une zone réactionnelle dite de reformage autotherme comprenant une chambre de combustion non catalytique et au moins un lit catalytique dans lequel débouchent les gaz issus de la chambre de combustion, la chambre de combustion utilisée devant alors permettre d'opérer à court temps de séjour et en défaut d'oxydant, comme cela est décrit dans la demande de brevet français FR-A-2.679.217.

Ainsi l'étape (a) du procédé selon l'invention est de préférence opérée en l'absence de catalyseur (cas de l'oxydation partielle), et à une température généralement comprise entre 1000 et 1500 ° C, de préférence entre 1200 et 1400 °C.

Lorsque l'étape (a) du procédé selon l'invention est opérée en présence d'un catalyseur, on emploie une vitesse volumétrique horaire (VVH) corrigée comprise entre 200 et 10000 h$^{-1}$, préférentiellement entre 400 et 8000 h$^{-1}$, où la VVH corrigée est égale à m x VVH, si m est le nombre moyen d'atomes de carbone de la charge.

On utilise alors préférentiellement un rapport molaire :
$$\frac{[H_2O + CO_2]}{\Sigma C} < 1,5$$
où $\Sigma C$ représente tout le carbone compris dans les hydrocarbures, et où $(H_2O+CO_2)$ représente la somme des débits d'eau et de $CO_2$ injectés.

Un préchauffage est conseillé, à la fois pour le combustible et pour l'oxydant avant leur introduction dans le réacteur. De préférence, le combustible peut être préchauffé entre 100 et 850 °C, tandis que l'oxydant peut subir un préchauffage compris entre 100 et 900 °C préférentiellement entre 200 et 750 °C.

La pression de la chambre de combustion est généralement comprise entre 0,1 et 15 MPa, de préférence entre 2 et 10 MPa (1 MPa = $10^6$ Pa).

L'étape de refroidissement est de préférence opérée par ajout d'eau ("quench"). Dans ce cas, l'eau ajoutée, en % poids par rapport à l'effluent à refroidir, est comprise entre 0,1 et 80, de préférence entre 1 et 15.

L'étape de transformation dans une zone de craquage de la majeure partie de l'effluent refroidi obtenu à l'étape précédente (étape (d) selon l'invention) ne nécessite généralement pas l'emploi d'un catalyseur.

Elle s'effectue à une température comprise entre 700 et 1300 °C de préférence entre 800 et 1100 °C. La pression n'a pas de valeur absolue puisque la perte de charge dans un réacteur de craquage atteint plusieurs bars (1 bar = $10^5$ Pa).

Une augmentation de pression défavorisant les réactions de craquage, il est habituel d'opérer à la plus basse pression compatible tant avec les pertes de charge qu'avec la pression utilisée dans l'étape suivante, soit par exemple entre 0,1 et 6 MPa, de préférence entre 1 et 4 MPa.

La transformation d'au moins la majeure partie de l'effluent pratiquement exempt d'eau obtenu à l'étape (e) par synthèse Fischer-Tropsch (étape (f) selon l'invention) est généralement opérée sous une pression totale habituellement comprise entre 0,1 et 15 MPa, et de préférence entre 0,5 et 10 MPa, la température étant généralement comprise entre 150 et 350 °C et de préférence entre 170 et 300 °C.

La vitesse volumétrique horaire est habituellement comprise entre 100 et 10000 volumes d'effluent par volume de catalyseur et par heure et de préférence entre 400 et 5000 volumes d'effluent par volume de catalyseur et par heure, et le rapport $H_2/CO$ dans le gaz de synthèse est habituellement compris entre 1:1 et 3:1, de préférence entre 1,2:1 et 2,5:1.

Le catalyseur utilisé pour ladite étape (f) peut être utilisé en poudre fine calibrée [10-700 μm (1 μm = $10^{-6}$ m) habituellement] ou en particules de diamètre équivalent compris généralement entre 2 et 10 mm, en présence d'une phase gazeuse, ou bien, dans les conditions opératoires, en présence d'une phase liquide et d'une phase gazeuse. La phase liquide peut être constituée par au moins un hydrocarbure ayant au moins 5 de préférence au moins 10 atomes de carbone par molécule. Ledit catalyseur est généralement à base d'au moins un métal des groupes 6 à 11, et contient préférentiellement au moins du cobalt ou du fer, dispersé sur un support à base d'au moins un oxyde métallique, préférentiellement la silice, l'alumine ou l'oxyde de titane. D'une manière préférée on utilise un catalyseur à base de cobalt tel que décrit dans la demande de brevet européen EP-A-0.581.619. Mais tout catalyseur connu de l'homme du métier est aussi envisageable.

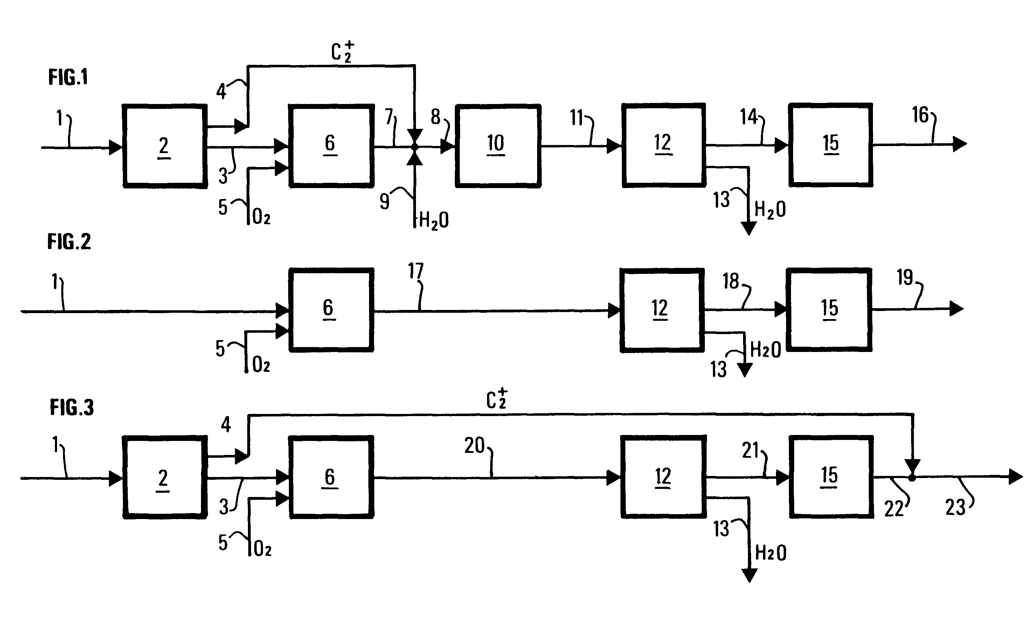

L'exemple 1 suivant non limitatif représente un mode de réalisation du procédé selon l'invention, qui peut être employé pour effectuer la conversion d'une charge comprenant essentiellement du gaz naturel en hydrocarbures essentiellement linéaires et saturés, et valoriser avantageusement les hydrocarbures $C_2^+$ essentiellement linéaires et saturés contenus dans ladite charge. Ledit exemple est illustré par la figure 1 ci-jointe.

EXEMPLE 1 (selon l'invention)

Du gaz naturel (1) comprenant 92 % de méthane et 8 % d'éthane (% exprimé en % carbone, c'est-à-dire en mole de carbone par mole de carbone contenu dans le gaz naturel introduit) est introduit dans une unité de séparation (2). En sortie de cette unité sont obtenus une première fraction (3) contenant essentiellement du méthane et une seconde fraction (4) contenant des hydrocarbures $C_2^+$ (essentiellement de l'éthane). Les compositions des différents effluents sont données dans le tableau 1 et le schéma de procédé est indiqué sur la figure 1.

La fraction riche en méthane (3) est introduite dans un réacteur d'oxydation partielle (6) où elle est convertie en gaz de synthèse (7) au moyen d'oxygène (5). Ledit réacteur est opéré à une température de 1260 °C et une pression de 3 MPa.

On ajoute alors au gaz de synthèse produit (7) la fraction (4) riche en éthane puis de l'eau (9), ce qui permet d'obtenir l'effluent (8), ce qui permet de diminuer la température de l'effluent issu de l'unité d'oxydation partielle ("quench") avant qu'il ne pénètre dans le réacteur de craquage (10).

Le craquage dudit effluent (8) est alors conduit à une pression de 3 MPa, une température d'entrée de 800 °C, et avec un temps de séjour de la charge dans le réacteur de craquage de 2,4 s. En sortie du réacteur (10), l'effluent (11) est à une température de 730 °C.

Après séparation de l'eau (13) dans l'unité de séparation (12), l'effluent essentiellement anhydre (14) est envoyé à l'unité de synthèse Fischer-Tropsch (15) fonctionnant également à 3 MPa, mais à une température de 240 °C et à une V.V.H. de 2000 $h^{-1}$, pour être converti en un mélange d'hydrocarbures (effluent 16) en présence d'un catalyseur comprenant 20% de cobalt, 0,8% de ruthénium, 0,2% de cuivre sur silice.

Le tableau 1 montre que l'association d'une étape d'oxydation partielle (6) et d'un craquage des hydrocarbures (10) permet d'obtenir un rapport $H_2/CO$ voisin de 2 en entrée du réacteur de synthèse Fischer-Tropsch, c'est-à-dire une valeur proche de la stoechiométrie des réactions de synthèse hydrocarbures.

D'autre part, l'étape de craquage permet de convertir pratiquement tout l'éthane essentiellement en éthylène (voir tableau 1, produit 11), lequel contribue à la synthèse d'hydrocarbures dans le réacteur Fischer-Tropsch.

Il en découle naturellement une productivité et une sélectivité élevées en hydrocarbures $C_5^+$ (soit respectivement 0,6228 moles d'hydrocarbures $C_5^+$ par mole de carbone introduite et 85,22 % d'hydrocarbures $C_5^+$).

EXEMPLE 2 : (comparatif)

Du gaz naturel (1) comprenant 92 % de méthane et 8 % d'éthane (% exprimé en % carbone) est introduit dans un réacteur d'oxydation partielle (6) où il est converti en gaz de synthèse (17) au moyen d'oxygène (5). Ledit réacteur est opéré à une température de 1260 °C et une pression de 3 MPa. Les compositions des différents effluents sont données dans le tableau 2 et le schéma de procédé est indiqué sur la figure 2. Le gaz de synthèse produit présente un rapport $H_2/CO$ égal à 1,21.

Après séparation de l'eau (13) dans l'unité de séparation (12), l'effluent essentiellement anhydre (18) est envoyé à l'unité de synthèse Fischer-Tropsch (15) fonctionnant également à 3 MPa, mais à une température de 240 °C et à une V.V.H. de 2000 $h^{-1}$, pour être converti en un effluent (19) (voir tableau 2). Le faible rapport $H_2/CO$ dans le gaz de synthèse anhydre (18) conduit à une productivité en $C_5^+$ nettement plus faible que celle de l'exemple 1 selon l'invention (0,4428 mole par mole de carbone introduit).

EXEMPLE 3 : (comparatif)

Un gaz naturel (1) comprenant 92 % de méthane et 8 % d'éthane (% exprimé en % carbone) est tout d'abord séparé en 2 fractions dans une unité de séparation (2). En sortie de cette unité sont obtenues une première fraction (3) contenant essentiellement du méthane et une seconde fraction (4) contenant des hydrocarbures $C_2^+$ (essentiellement de l'éthane). Les compositions des différents effluents sont données dans le tableau 3 et le schéma de procédé est indiqué sur la figure 3.

La fraction riche en méthane (3) est introduite dans un réacteur d'oxydation partielle (6) où elle est convertie en gaz de synthèse (20) au moyen d'oxygène (5). Ledit réacteur est opéré à une température de 1260 °C et une pression de 3 MPa.

Le gaz de synthèse produit (20) présente un rapport $H_2$/CO égal à 1,88. L'effluent (21) obtenu après séparation de l'eau (13) dans l'unité de séparation (12) est introduit dans le réacteur de synthèse Fischer-Tropsch (15) où il est transformé en un effluent (22), auquel on ajoute l'effluent de la ligne (4) pour obtenir l'effluent final (23) (tableau 3), l'unité de synthèse Fischer-Tropsch fonctionnant à 3 MPa, 240 °C, et avec une V.V.H. de 2000 h$^{-1}$. La productivité et la sélectivité en hydrocarbures $C_5^+$ obtenues sont nettement plus faibles que celles de l'exemple 1 selon l'invention.

| Effluent | 1 | 3 | 4 | 7 | 8 | 11 | 14 | 16 | Sélectivité (%C) |
|---|---|---|---|---|---|---|---|---|---|
| $CH_4$ | 0,92 | 0,92 | 0 | 0,0089 | 0,0089 | 0,0134 | 0,0134 | 0,0467 | 6,39 |
| $C_2H_4$ | 0 | 0 | 0 | 0 | 0 | 0,0751 | 0,0751 | | |
| $C_2H_6$ | 0,08 | 0 | 0,08 | 0 | 0,0784 | 0,0001 | 0,0001 | 0,0240 | 3,28 |
| $C_3-C_4$ | 0 | 0 | 0 | 0 | 0 | 0,0003 | 0,0003 | | |
| $C_5^+$ | 0 | 0 | 0 | 0 | 0 | 0 | 0 | 0,6228 | 85,22 |
| $H_2$ | 0 | 0 | 0 | 1,6581 | 1,6609 | 1,7321 | 1,7321 | 0,2533 | - |
| $CO$ | 0 | 0 | 0 | 0,8822 | 0,8837 | 0,8808 | 0,8808 | 0,1938 | - |
| $CO_2$ | 0 | 0 | 0 | 0,0289 | 0,0289 | 0,0304 | 0,0304 | 0,0373 | 5,10 |
| $H_2O$ | 0 | 0 | 0 | 0,1640 | 1,4679 | 1,4595 | 0 | 0,6732 | - |
| $H_2/CO$ | - | - | - | 1,88 | 1,88 | 1,97 | 1,97 | 1,31 | - |

TABLEAU 1 : Compositions des effluents de l'exemple 1 exprimées en moles de carbone par mole de carbone dans le gaz naturel introduit.

EP 0 679 709 B1

TABLEAU 2:

| Compositions des effluents de l'exemple 2 exprimées en moles de carbone par mole de carbone dans le gaz naturel introduit. | | | | | |
|---|---|---|---|---|---|
| **Effluent** | **1** | **1 7** | **1 8** | **1 9** | **Sélectivité (°C)** |
| $CH_4$ | 0,92 | 0 | 0 | 0,0195 | 3,58 |
| $C_2H_4$ | 0 | 0 | 0 | | |
| $C_2H_6$ | 0,08 | 0 | 0 | 0,0097 | 1,79 |
| $C_3$-$C_4$ | 0 | 0 | 0 | | |
| $C_5^+$ | 0 | 0 | 0 | 0,4428 | 81,54 |
| $H_2$ | 0 | 1,2262 | 1,2262 | 0,2275 | - |
| C O | 0 | 1,0138 | 1,0138 | 0,5272 | - |
| $CO_2$ | 0 | 0,0662 | 0,0662 | 0,0711 | 13,08 |
| $H_2O$ | 0 | 0,8538 | 0,8538 | 1,3210 | - |
| $H_2$/CO | - | 1,21 | 1,21 | 0,43 | - |

| Effluent | 1 | 3 | 4 | 2 0 | 2 1 | 2 2 | 2 3 | Sélectivité (%C) |
|---|---|---|---|---|---|---|---|---|
| $CH_4$ | 0,92 | 0,92 | 0 | 0,0089 | 0,0089 | 0,0612 | 0,0612 | 7,85 |
| $C_2H_4$ | 0 | 0 | 0 | 0 | 0 | | | - |
| $C_2H_6$ | 0,08 | 0 | 0,08 | 0 | 0 | 0,0232 | 0,1032 | 13,23 |
| $C_3$-$C_4$ | 0 | 0 | 0 | 0 | 0 | | | - |
| $C_5^+$ | 0 | 0 | 0 | 0 | 0 | 0,5796 | 0,5796 | 74,36 |
| $H_2$ | 0 | 0 | 0 | 1,6581 | 1,6581 | 0,2338 | 0,2338 | - |
| $CO$ | 0 | 0 | 0 | 0,8822 | 0,8822 | 0,2206 | 0,2206 | - |
| $CO_2$ | 0 | 0 | 0 | 0,0289 | 0,0289 | 0,0355 | 0,0355 | 4,55 |
| $H_2O$ | 0 | 0 | 0 | 0,1640 | 0 | 0,8125 | 0,8125 | - |
| $H_2/CO$ | - | - | - | 1,88 | 1,88 | 1,06 | 1,06 | - |

TABLEAU 3 : Compositions des effluents de l'exemple 3 exprimées en moles de carbone par mole de carbone dans le gaz naturel introduit.

EP 0 679 709 B1

**Revendications**

1. Procédé de préparation d'hydrocarbures essentiellement linéaires et saturés à partir d'une charge comprenant principalement du gaz naturel, ledit procédé comprenant les étapes successives suivantes, les étapes (b) et (c) étant telles qu'elles peuvent être réalisées soit simultanément, soit dans l'ordre (b) puis (c), soit dans l'ordre (c) puis (b) :

   (a) la production de gaz de synthèse par traitement d'au moins une partie de ladite charge permettant l'obtention d'un effluent comprenant principalement de l'oxyde de carbone, de l'hydrogène et éventuellement du dioxyde de carbone,

   (b) le mélange d'un effluent comprenant au moins la majeure partie de l'effluent obtenu à l'étape précédente et d'un effluent comprenant principalement au moins un hydrocarbure comportant de 2 à 4 atomes de carbone par molécule,

   (c) le refroidissement d'un effluent comprenant au moins la majeure partie du mélange obtenu à l'étape précédente, permettant l'obtention d'un effluent refroidi,

   (d) la transformation dans une zone de craquage à une température comprise entre 700°C et 1300°C et une pression comprise entre 0,1 et 6 MPa de la majeure partie de l'effluent refroidi obtenu à l'étape précédente, permettant l'obtention d'un effluent comprenant des hydrocarbures insaturés,

   (e) la séparation d'au moins la majeure partie de l'effluent obtenu à l'étape (d) en un effluent comprenant principalement de l'eau et en un effluent pratiquement exempt d'eau,

   (f) la transformation d'au moins la majeure partie de l'effluent pratiquement exempt d'eau obtenu à l'étape (e) par synthèse Fischer-Tropsch à une température comprise entre 150°C et 350°C et une pression comprise entre 0,1 et 15 MPa, en présence d'un catalyseur de synthèse Fischer-Tropsch, permettant d'obtenir l'effluent final.

2. Procédé selon la revendication 1 dans lequel l'effluent de l'étape (b) comprenant principalement au moins un hydrocarbure comportant de 2 à 4 atomes de carbone par molécule est au moins partiellement issu de l'un au moins des procédés suivants : un procédé de synthèse Fischer-Tropsch, un procédé d'hydrotraitement.

3. Procédé selon l'une des revendications 1 ou 2 dans lequel on sépare l'effluent de l'étape (f) en un premier effluent contenant principalement des hydrocarbures essentiellement linéaires et saturés contenant de 2 à 4 atomes de carbone par molécule et en un second effluent contenant principalement des hydrocarbures essentiellement linéaires et saturés contenant au moins cinq atomes de carbone par molécule.

4. Procédé selon la revendication 3 dans lequel l'effluent comprenant principalement au moins un hydrocarbure $C_2$-$C_4$ de l'étape (b) comprend au moins la majeure partie dudit premier effluent obtenu à l'issue de l'étape (f).

5. Procédé selon l'une des revendications 3 ou 4 dans lequel on procède, à l'issue de l'étape (f), à l'hydrotraitement dudit second effluent.

6. Procédé selon la revendication 5 dans lequel l'effluent défini à l'étape (b) comprenant principalement au moins un hydrocarbure C2-C4 comprend au moins la majeure partie d'un effluent comprenant principalement des hydrocarbures C2-C4 issus de l'hydrotraitement.

7. Procédé selon l'une des revendications 1 à 6 dans lequel, préalablement à l'étape (a) du procédé selon l'invention, on procède à une séparation de la charge en une première fraction enrichie en méthane et une seconde fraction enrichie en au moins un hydrocarbure comprenant de 2 à 4 atomes de carbone par molécule, de façon à ce que la partie de ladite charge traitée à l'étape (a) soit principalement la majeure partie de ladite première fraction.

8. Procédé selon la revendication 7 dans lequel l'effluent comprenant principalement au moins un hydrocarbure C2-C4 de l'étape (b) comprend au moins la majeure partie de ladite seconde fraction.

9. Procédé selon l'une des revendications 1 à 8 dans lequel l'étape a) est réalisée au moyen d'un procédé d'oxydation partielle.

10. Procédé selon l'une des revendications 1 à 9 dans lequel on prépare des hydrocarbures essentiellement linéaires et saturés comprenant une proportion importante d'hydrocarbures comprenant au moins 5 atomes de carbone par molécule.

**Patentansprüche**

1. Verfahren zur Herstellung von im wesentlichen linearen und gesättigten Kohlenwasserstoffen ausgehend von einer hauptsächlich Erdgas enthaltenden Charge, wobei das Verfahren die folgenden aufeinanderfolgenden Stufen umfasst, wobei die Stufen (b) und (c) als solche entweder gleichzeitig oder in der Reihenfolge (b), dann (c) oder in der Reihenfolge (c), dann (b) verwirklicht werden können:

   (a) die Herstellung von Synthesegas durch Behandlung von wenigstens einem Teil dieser Charge, die den Erhalt eines Abstroms ermöglicht, der hauptsächlich Kohlenstoffoxid, Wasserstoff und gegebenenfalls Kohlendioxid umfasst,

   (b) die Mischung eines Abstroms, der wenigstens den größeren Teil des bei der vorangehenden Stufe erhaltenen Abstroms umfasst und eines Abstroms, der hauptsächlich wenigstens einen Kohlenwasserstoff mit 2 bis 4 Kohlenstoffatomen pro Molekül umfasst,

   (c) die Abkühlung eines wenigstens den größeren Teil der bei der vorangehenden Stufe erhaltenen Mischung umfassenden Abstroms, was den Erhalt eines abgekühlten Abstroms ermöglicht,

   (d) die Umwandlung des größeren Teils des abgekühlten Abstroms, der bei der vorangehenden Stufe erhalten wurde, in einer Crackzone bei einer Temperatur, die zwischen 700°C und 1300°C liegt, und einem Druck, der zwischen 0,1 und 6 MPa liegt, was den Erhalt eines Abstroms ermöglicht, der ungesättigte Kohlenwasserstoffe umfasst,

   (e) die Trennung wenigstens des Hauptteils des bei Stufe (d) erhaltenen Abstroms in einen Abstrom, der hauptsächlich Wasser umfasst und einen Abstrom, der praktisch wasserfrei ist,

   (f) die Umwandlung von wenigstens dem größeren Teil des praktisch wasserfreien, bei Stufe (e) erhaltenen Abstroms durch Fischer-Tropsch Synthese bei einer Temperatur, die zwischen 150°C und 350°C liegt und einem Druck, der zwischen 0,1 und 15 MPa liegt, in Gegenwart eines Katalysators zur Fischer-Tropsch Synthese, was es ermöglicht, den Endabstrom zu erhalten.

2. Verfahren nach Anspruch 1, bei dem der Abstrom der Stufe (b), der hauptsächlich wenigstens einen Kohlenwasserstoff umfasst, der 2 bis 4 Kohlenstoffatome pro Molekül aufweist, wenigstens teilweise aus wenigstens einem der Folgenden Verfahren stammt: ein Fischer-Tropsch Syntheseverfahren, ein Hydrotreatmentverfahren.

3. Verfahren nach einem der Ansprüche 1 oder 2, bei dem man den Abstrom der Stufe (f) in einen ersten, hauptsächlich im wesentlichen lineare und gesättigte, 2 bis 4 Kohlenstoffatome pro Molekül umfassende Kohlenwasserstoffe enthaltenden Abstrom und einen zweiten, hauptsächlich im wesentlichen lineare und gesättigte, fünf Kohlenstoffatome pro Molekül umfassende Kohlenwasserstoffe enthaltenden Abstrom trennt.

4. Verfahren nach Anspruch 3, bei dem der Abstrom, der hauptsächlich wenigstens einen $C_2$-$C_4$ Kohlenwasserstoff der Stufe (b) umfasst, wenigstens den größeren Teil dieses ersten ausgehend von Stufe (f) erhaltenen Kohlenwasserstoffs umfasst.

5. Verfahren nach einem der Ansprüche 3 oder 4, bei dem man am Ende von der Stufe (f) zu einem Hydrotreatment dieses zweiten Abstroms übergeht.

6. Verfahren nach Anspruch 5, bei dem der bei Stufe (b) festgelegte Abstrom, der hauptsächlich wenigstens einen $C_2$-$C_4$ Kohlenwasserstoff umfasst, wenigstens den größeren Teil eines hauptsächlich aus dem Hydrotreatment stammenden, $C_2$-$C_4$ Kohlenwasserstoffe umfassenden Abstroms umfasst.

7. Verfahren nach einem der Ansprüche 1 bis 6, bei dem man vor der Stufe (a) des Verfahrens nach der Erfindung zu einer Trennung der Charge in eine erste an Methan angereicherte Fraktion und eine zweite an wenigstens einem 2 bis 4 Kohlenstoffatomen pro Molekül umfassenden Kohlenwasserstoff angereicherte Fraktion derart verfährt, dass der bei Stufe (a) behandelte Teil der Charge hauptsächlich der größere Teil der ersten Fraktion ist.

8. Verfahren nach Anspruch 7, bei dem der hauptsächlich wenigstens einen $C_2$-$C_4$ Kohlenwasserstoff der Stufe (b) umfassende Abstrom wenigstens den größeren Teil der zweiten Fraktion umfasst.

9. Verfahren nach einem der Ansprüche 1 bis 8, bei dem die Stufe a) mittels eines partiellen Oxidationsverfahrens verwirklicht wird.

10. Verfahren nach einem der Ansprüche 1 bis 9, bei dem man im wesentlichen lineare und gesättigte Kohlenwasserstoffe herstellt, die einen beträchtlichen Anteil von Kohlenwasserstoffen umfassen, die wenigstens 5 Kohlenstoffatome pro Molekül umfassen.

## Claims

1. A process for the preparation of essentially linear and saturated hydrocarbons from a feed containing mainly natural gas, said process comprising the following successive steps, steps (b) and (c) being capable of being carried out either simultaneously or in the order (b) then (c) or in the order (c) then (b):

   (a) producing synthesis gas by treating at least a portion of said feed to obtain an effluent containing mainly carbon monoxide, hydrogen and optionally carbon dioxide,
   (b) mixing an effluent comprising at least a major portion of the effluent obtained from the preceding step with an effluent comprising mainly at least one hydrocarbon containing 2 to 4 carbon atoms per molecule,
   (c) cooling an effluent comprising at least the major portion of the mixture obtained from the preceding step, to produce a cooled effluent,
   (d) transforming the major portion of the cooled effluent from the preceding step in a cracking zone, to obtain an effluent comprising unsaturated hydrocarbons,
   (e) separating at least a major portion of the effluent from step (d) into an effluent comprising mainly water and an effluent which is practically free of water,
   (f) transforming at least a major portion of the practically water-free effluent from step (e) by a Fischer-Tropsch synthesis, at a temperature comprised between 150°C and 250°C and a pressure comprised between 0.1 and 15 MPa, in the presence of a Fischer-Tropsch synthesis catalyst to produce the final effluent.

2. A process according to claim 1, wherein the effluent from step (b) comprising mainly at least one hydrocarbon containing 2 to 4 carbon atoms per molecule is produced at least partially from at least one of the following processes: a Fischer-Tropsch synthesis process, or a hydrotreatment process.

3. A process according to claim 1 or claim 2, wherein the effluent from step (f) is separated into a first effluent containing mainly essentially linear and saturated hydrocarbons containing 2 to 4 carbon atoms per molecule and a second effluent containing mainly essentially linear and saturated hydrocarbons containing at least five carbon atoms per molecule.

4. A process according to claim 3, wherein the effluent comprising mainly at least one $C_2$-$C_4$ hydrocarbon from step (b) comprises at least a major portion of said first effluent obtained from step (f).

5. A process according to claim 3 or claim 4, wherein hydrotreatment of said second effluent is carried out after step (f).

6. A process according to claim 5, wherein the effluent defined in step (b) comprising mainly at least one $C_2$-$C_4$ hydrocarbon comprises at least a major portion of an effluent comprising mainly $C_2$-$C_4$ hydrocarbons from the hydrotreatment step.

7. A process according to any one of claims 1 to 6, wherein, prior to step (a) of the process of the invention, the feed is separated into a first methane-enriched fraction and into a second fraction which is enriched in at least one hydrocarbon containing 2 to 4 carbon atoms per molecule, such that the portion of said feed treated at step (a) is mainly the major portion of said first fraction.

8. A process according to claim 7, wherein the effluent containing mainly at least one $C_2$-$C_4$ hydrocarbon from step (b) comprises at least a major portion of said second fraction.

9. A process according to any one of claims 1 to 8, wherein step a) is performed by using a partial oxidation process.

10. A process according to any one of claims 1 to 9, wherein essentially linear and saturated hydrocarbons are prepared which contain a large proportion of hydrocarbons containing at least 5 carbon atoms per molecule.

FIG.1

FIG.2

FIG.3